# EUROPEAN PATENT APPLICATION

(11) **EP 1 026 236 A1**
(43) Date of publication of application: **09.08.2000**
(21) Application number: 99200362.4
(22) Date of filing: 04.02.1999
(51) Int. Cl.: C12N 9/02, C12P 1/00, C12P 17/18, C12P 35/00, C12P 37/00, C12N 1/20

(54) **Auxotrophic strains for secondary metabolite biosynthesis**

(71) Applicant: DSM N.V., 6411 TE Heerlen (NL)
(72) Inventor: Streekstra, Hugo, 1017 SP Amsterdam (NL); Koekman, Bertus Pieter, 2636 BG Schipluiden (NL); De Laat, Wilhelmus Theodorus Antonius Maria, 4817 KW Breda (NL)
(74) Representative: Visser-Luirink, Gesina, Dr.

(57) **Abstract**

The present invention provides auxotrophic strains for secondary metabolite biosynthesis and a novel fermentation process for the overproduction of β-lactams or other secondary metabolites by these lipoate and/or thiamine auxotrophic strains which show altered activity in pyruvate dehydrogenase and/or α-ketoglutarate dehydrogenase.

## Description

### Field and background of the invention

The present invention relates to mutant microorganisms with an increased production level of secondary metabolites, and to an improved fermentation process for the production of secondary metabolites.

The microbial production of β-lactams for use in health care or feed-products has a long history of more than 50 years. One of the first examples was the production of penicillin starting in the Second World War. Over the years, microbial strains producing penicillins and many other anti-bacterials and antifungals have been screened for in nature and have successively been improved traditionally by classical strain improvement, which is characterised by random mutagenesis using UV and/or chemical mutagenic agents like NTG and selection for higher production levels in agar plates and/or submerged cultures.

A next step made in the '60s and '70s was to deregulate microorganisms for their metabolic repression by selecting in a rational way for resistance to metabolic analogues and thereby increasing overproduction. These manipulations were directed at regulatory aspects of the cell.

Nowadays, since production of β-lactams is very time consuming, there is still an urgent need for strains or methods to further increase production yield.

Surprisingly, it was found that a higher production level of β-lactams or other secondary metabolites could be achieved by using lipoate or thiamine or double auxotrophic mutants. Among these auxotrophic mutants, organisms occur which are overproducing pyruvate and/or α-ketoglutarate (αKG) which, under specific fermentation conditions, may result in increased production of β-lactams.

So far, using lipoate auxotrophic strains, only overproduction of primary metabolites such as amino acids or organic acids has been achieved [Yokato, A., *et al.,* Agric. Biol. Chem. 53 (1989) 2037-2044]. However, nowhere it was shown or even suggested that the use of lipoate auxotrophic strains was favourable in the preparation of secondary metabolites in high yield.

### Summary of the invention

The present invention relates to mutant microorganisms which are auxotroph for lipoate or for thiamine or for both. Furthermore, said mutant strains of Streptomyces sp. or Penicillium sp. or Acremonium sp. show an attenuated α-ketoglutarate dehydrogenase (KGDH) or pyruvate dehydrogenase activity (PDH), and are characterised in that they are able to produce secondary metabolites at a higher level as compared to wild-type strains. These auxotrophic microorganisms are isolated via a method well known in the art.

Also mutated strains which are for another reason attenuated in pyruvate dehydrogenase (PDH)- and α-ketoglutarate dehydrogenase (KGDH) activity may show highly increased production of secondary metabolites via the same principle as described for the auxotrophic strains.

In another embodiment of the invention we describe a specific fermentation process which enables highly increased β-lactam (i.e clavulanic acid, a penicillin, or a cephalosporin) or other secondary metabolite production by making use of a specific fermentation process wherein the efficiency of the reactions catalysed by pyruvate dehydrogenase (PDH) and α-ketoglutarate dehydrogenase (KGDH) respectively is effectively manipulated by adjusting the feed of the co-factors lipoate and/or thiamine depending on the level of co-factor bradytrophy of each specific mutant.

### Detailed description of the invention

Many secondary metabolites are produced from glucose or other low-cost carbon and energy sources such as fructose, sucrose, maltose, lactose, or polysaccharides like starch, maltodextrins and inulin (or other fructose polymers), nitrogen sources, primarily proteins and (partially) hydrolysed proteins such as vegetable flours, grasses such as those useful in fermentation industry (i.e. soybean flour, linseed flour, peanut flour, potato flour, sunflower, pea- or bean flour, cotton seed flour, wheat gluten, whole wheat, rice meal), or peptides derived from animal or plant sources called peptones, or proteins derived from microbial sources like yeast extracts, triglycerides such as soybean oil, sun flour oil, olive oil, tri-oleate etc., (poly-) alcohols such as ethanol, propanol, glycerol, mannitol, or organic acids or a salt thereof such as acetate, propionate, succinate, fumarate, malate, adipate, citrate, lactate, gluconate etc.

However looking at the intermediary metabolism, the actual starting molecules for biosynthesis (anabolism) are derived from intermediates with a lower energy content (catabolites) of the carbon source used either derived from the glycolysis ending up in pyruvate, or from the Tricarboxylic acid cycle (TCA-cycle).

Classical improvement of industrial strains will aim at amplification of the biosynthetic pathway leading to the desired product. Most efforts into molecular genetic strain improvement have also focused on the genes and enzymes of this pathway. However, in addition to this, there is a need to generate the building blocks to fuel the pathway, and the co-substrates required for its optimal activity, at a sufficient rate. As a third requirement for optimal production, it is necessary to channel the building blocks and co-substrates preferentially to the pathway of interest, sometimes at the expense of competing metabolic pathways.

In general, accumulation of these intermediates (such as pyruvate or α-ketoglutarate) can be achieved in various ways:
1. Lowering the expression of enzymes which decarboxylate the above mentioned intermediates by genetic techniques,
2. Inhibiting these decarboxylating enzymes by using chemicals [Humphries, K.M., *et al*., Biochemistry 37 (1998) 15835-15841],
3. Using a substrate, or a cheap cosubstrate, which is easily converted to pyruvate or α-KG. Such (co-)substrates could be citric acid (2 steps to αKG in TCA), lactic acid (one step to pyruvate), glutamate (1 step to αKG), any other TCA-intermediate combined with a source of non-pyruvate derived acetyl-CoA (like vegetable oils, or other compounds catabolised by β-oxidation, or ethanol or acetate). An elegant example is adipate as co-substrate yielding succinyl-CoA and acetyl-CoA which can be converted via the TCA-cycle to αKG.
4. Any combination of the options mentioned above.

The lactams and clavams are derivatives of amino acids, which are essential for various other cellular process, and ultimately for growth. The primary intermediates pyruvate and α-KG are among the most central metabolites in cellular biochemistry.

Hence, there are complicated and often poorly understood, relationships between overproduction of the desired molecule on the one hand, and optimal functioning of the cell on the other hand. In general, one may assume that one should favor the product formation in its competition with other cellular processes for essential intermediates, but not excessively so, to avoid negative effects on growth and viability. Therefore, striking the right balance between the formation of bio-mass and the desired product is of paramount importance, both for process economical and microbial physiological reasons. Although this general principle will be widely recognized, the way to achieve this balance is by no means a straightforward approach.

Furthermore, although both β-lactams and clavams are amino acid derivatives, penicillins (PenV and PenG) and cephalosporins are primarily condensation products of three amino acids (aminoadipic acid, cysteine and valine), whereas clavulanic acid is derived from a single amino acid (arginine) which undergoes extensive chemical modification to arrive at the cyclic lactam structure. Therefore, it cannot be predicted whether a production strategy that is effective in one case would also be effective for other products.

In the present invention we disclose lipoate and/or thiamine auxotrophic strains which in the underlying invention also includes partially auxotrophic i.e. bradytrophic strains (Penicillium sp. such as P. chrysogenum, Streptomyces sp. such as S. clavuligerus, and Acremonium sp. such as A. chrysogenum) with attenuated Pyruvate Dehydrogenase (PDH)- and α-Ketoglutarate Dehydrogenase (KGDH) activity. These mutant strains are characterised in that they produce secondary metabolites at higher level as compared to wild-type strains. These auxotrophic microorganisms are isolated via a method well known in the art, which consists essentially of enrichment of a mutagenised population of lipoate- and/or thiamine-requiring microorganisms by selectively killing prototrophs and selecting said mutants by replica plating.

Furthermore, mutated strains wherein said auxotrophy has reverted to prototrophy might retain their high production capacity for secondary metabolites. Therefore, these reverted mutants are also part of the underlying invention.

Another aspect of the invention discloses a highly increased β-lactam production (notably penicillins and cephalosporins), and clavam production (in particular clavulanic acid), by making use of a specific fermentation process in which the efficiency of the reactions catalysed by Pyruvate Dehydrogenase (PDH) and α-Ketoglutarate Dehydrogenase (αKGDH) respectively is effectively manipulated.

Of course, the recovery of the impure clavulanic acid solution as formed by the fermentation in a fed batch, continuous or semi-continuous mode using the recombinant microorganisms of the underlying invention, as well as the subsequent conversion thereof into a pharmaceutically acceptable salt thereof, preferably potassium clavulanate, via an amine salt of clavulanic acid, do form an aspect of the present invention. One of the most advantageous procedures is the conversion of the impure clavulanic acid into an amino salt thereof by adding the corresponding amino salt forming compound as for instance N,N,N',N'-tetramethylethylenediamine, 1,3-bis(di-methylamino)-2-propanol, t-butylamine, t-octylamine, benzhydrylamine and bis (2-(dimethyl-amino)ethyl)ether and reacting said amine clavulanate with a non-toxic pharmaceutically acceptable salt as for instance potassium ethylhexanoate to form the corresponding purified salt, for instance potassium clavulanate.

Also, the recovery of other β-lactams produced by the mutant microorganisms of underlying invention, such as Penicillin G and Penicillin V, and optional conversion of said β-lactams into key intermediates (such as 6-APA or 7-ADCA) and subsequently into amoxycillin, ampicillin, cephadroxil or cephalexin or other pharmaceutical acceptable compounds via methods well known in the art see international applications WO 95/04148 and WO 98/48039, do form an aspect of the present invention.

The following example is only to be considered as illustration of the present invention, and is not to be construed as limiting.

### Example 1

Overproduction of clavulanic acid by mutants of Streptomyces clavuligerus low in PDH/αKGDG activity.

Streptomyces clavuligerus ATCC 27064 or a strain derived thereof by mutation and selection for higher clavulanic acid productivity, was mutagenized with nitrosoguanidine by methods well known in the art.

Ca. 10⁸ mutagenized spores were inoculated into a minimal medium containing (g/l): (NH₄)₂SO₄, 2; L-asparagine, 9; L-arginine, 2; glycerol, 50; maltose.2H₂O, 6; Na₂-succinate.6H₂O, 5.5; KH₂PO₄, 1.5; MgSO₄.7H₂O, 0.5; CaCl₂.2H₂O, 0.01; FeSO₄.7H₂O, 0.25; MnCl₂.4H₂O, 0.01; ZnSO₄.7H₂O, 0.01; NiSO₄.6H₂O, 0.01; 3-(N-morpholino)-propanesulfonic acid, 21; trace elements solution 1 (H₂SO₄, 20.4; citric acid.H₂O, 50; ZnSO₄.7H₂O, 16.75; CUSO₄.5H₂O, 2.5; MnCl₂.4H₂O, 1.5; H₃BO₃, 2; Na₂MoO₄.2H₂O, 2), 0.06 (ml/I), pH 6.8, and incubated at 28°C.

After germination of the spores, the mycelia were removed from the medium by filtration. The filtrate, which contains ungerminated spores and mycelial fragments, was then treated with pentachlorophenol according to the method of Ganju and lyengar (Hindustan Antibiot. Bull. (1968) 11: 12-21) to selectively kill the mycelial fragments.

After washing out the pentachlorophenol, the ungerminated spores were inoculated into fresh minimal medium supplemented with 50 pg/ml of either lipoic acid or thiamine hydrochloride and allowed to grow out at 28 °C. The culture was then plated in its entirety on sporulation agar containing (g/l): glucose, 10; casein hydrolysate, 2; yeast extract, 1; beef extract, 1; trace elements solution 2 (ZnSO₄.7H₂O, 2.8; Ferric ammonium citrate, 2.7; CuSO₄.5H₂O, 0.125; MnSO₄.H₂O, 1; CoCl₂.6H₂O, 0.1; Na₂B₄O₇.10H₂O, 0.16; Na₂MoO₄.2H₂O, 0.054), 5 (ml/l), agar, 20 (pH 7.3) and incubated at 28 °C.

After sporulation had occurred, the spores were collected, plated on minimal medium (*vide supra*), supplemented with 50 µg/ml of either lipoic acid or thiamine hydrochloride and solidified with 2% agar, and subsequently replicaplated on minimal medium without vitamin supplements.

Auxotrophic mutants were then identified and individually inoculated into a seed medium containing (g/l): dextrin, 20; pharmamedia, 25; polypeptone, 5; yeast extract, 2.5; CoCl₂. 6 H₂O, 0.002; FeSO₄. 7 H₂O, 0.04; CaCO₃, 1 (pH before sterilisation 7.5).

The cultures were incubated in an orbital shaker at 26-28 °C, 220-250 rpm, for 24 hours and used to inoculate 20 volumes of production medium containing (g/l): soybean meal, 17.5; casein hydrolysate, 21.9; glycerol, 52.5; KH₂PO₄, 1.35; MgSO₄. 7 H₂O, 0.58; CaCl₂. 2 H₂O, 0.31; FeSO₄. 7 H₂O, 0.44; trace elements solution 1 (vide supra), 1.75 (ml/l); 3-(N-morpholino)-propane-sulfonic acid, 10.5; SAG 5693 (Basildon), 1 (pH before sterilisation 7.0).

The incubations were then continued for 120 hours. Clavulanic acid was produced in high yield, as assayed in the culture filtrate at the end of the fermentation by HPLC methods well known in the art.

## Claims

1. A mutant microorganism, characterised in that said microorganism is auxotrophic for lipoate and/or thiamine and has an increased production level of secondary metabolites compared to the corresponding wild-type microorganism.

2. A mutant microorganism, characterised in that said microorganism is attenuated in α-ketoglutarate dehydrogenase or pyruvate dehydrogenase and has an increased production level of secondary metabolites compared to the corresponding wild-type microorganism.

3. A mutant microorganism according to claim 1 or 2, wherein said microorganism has an increased production level of β-lactams.

4. A mutant microorganism according to anyone of claims 1-3, wherein said microorganism is a Streptomyces species, Penicillium chrysogenum, or Acremonium chrysogenum.

5. A mutant microorganism according to claim 3 or 4, wherein said β-lactam is clavulanic acid, a penicillin or a cephalosporin.

6. A process to isolate mutant microorganisms of anyone of claims 1-5, wherein said process consists essentially of enrichment of a mutagenised population of lipoate- and/or thiamine-requiring microorganisms by selectively killing prototrophs and selecting said mutants by replica plating.

7. A process for the production of secondary metabolites by fermentation of mutant microorganisms of anyone of the claims 1-5 in a fermentation medium wherein the concentration of the co-factors lipoate and/or thiamine is effectively manipulated depending on the level of co-factor bradytrophy of each specific mutant, and subsequent recovery of said secondary metabolites by isolation.

8. A process according to claim 7 in which the fermentation is carried out in a fed batch, a continuous or semi-continuous mode.

9. A process according to claim 7 or 8, wherein said secondary metabolite is clavulanic acid and subsequent conversion to a pharmaceutically acceptable salt thereof, by converting impure clavulanic acid into amine clavulanate by adding the corresponding amine salt forming compound and reacting said amine clavulanate with a non-toxic pharmaceutically acceptable salt to form the corresponding purified salt of clavulanic acid.

10. A process according to claim 7 or 8, wherein said secondary metabolite is a penicillin or cephalosporin, and subsequent conversion thereof into key intermediates and/or pharmaceutical acceptable compounds.
